(19) Europäisches Patentamt
European Patent Office
Office européen des brevets

(11) **EP 4 675 636 A1**

(12) **EUROPEAN PATENT APPLICATION**

(43) Date of publication:
**07.01.2026 Bulletin 2026/02**

(21) Application number: **24185586.5**

(22) Date of filing: **01.07.2024**

(51) International Patent Classification (IPC):
**G16H 40/63** (2018.01)

(52) Cooperative Patent Classification (CPC):
**G16H 40/63;** A61B 5/746; G16H 50/30

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **BIST, Cambodge**
**Eindhoven (NL)**

(74) Representative: **Philips Intellectual Property & Standards**
**High Tech Campus 52**
**5656 AG Eindhoven (NL)**

(54) **ALARM THRESHOLDS FOR A PHYSIOLOGICAL PARAMETER**

(57) A mechanism for predicting whether or not an alarm threshold would benefit from being updated, i.e., is out of date. The physiological parameter, associated with the alarm threshold, is monitored and a set of values of the physiological parameter is processed to produce a proximity measure. Responsive to the proximity measure meeting one or more certain criteria, an update indicator indicating the alarm threshold should be updated is produced.

FIG. 2

**Description**

FIELD OF THE INVENTION

The present invention relates to the field of medical monitoring, and in particular to alarm thresholds for use in medical monitoring.

BACKGROUND OF THE INVENTION

There is an increasing use of medical monitoring devices within the medical field for automated monitoring of a (medical) subject. Typically, such devices will monitor the value of a physiological parameter and generate an alarm, particularly a user-perceptible alarm, if the value breaches an alarm threshold. It is common for each monitored physiological parameter to be associated with two alarm thresholds, namely an upper and lower alarm threshold.

Examples of physiological parameters are well known in the art, and include any known vital sign or other physiological parameter. A non-exhaustive list of example physiological parameters include: SpO2; heart rate; respiratory rate; blood pressure; body temperature; blood glucose level; concentration of blood constituents (e.g., $HBO_2$ or HB) and so on.

It has been recognized that an inaccurate or incorrect alarm threshold can lead to deterioration of the (medical) subject being overlooked or missed. This can have serious consequences for the subject. There is therefore a desire to ensure that alarm thresholds are correct and/or appropriate for the subject.

SUMMARY OF THE INVENTION

The invention is defined by the claims.

According to examples in accordance with an aspect of the invention, there is provided a computer-implemented method for determining whether to adjust an alarm threshold for a physiological parameter.

The computer-implemented method comprises: monitoring a physiological parameter to determine a measure of proximity between a set of values of the physiological parameter and the alarm threshold for the physiological parameter; and responsive to the measure of proximity meeting one or more predetermined criteria, generating an update indicator that indicates that it is recommended to update the alarm threshold.

The present disclosure provides a mechanism for suggesting or indicating when an alarm threshold is inappropriate or outdated. It is proposed to determine a measure of proximity between values of a physiological parameter and an alarm threshold. This measure of proximity is used to determine whether or not the alarm threshold is appropriate (e.g., for a specific medical subject).

By way of example, if a measure of proximity indicates that the values are distant from the alarm threshold, then this may indicate that the alarm threshold is too far away, meaning that significant deviation of the value(s) of the physiological parameter, indicative of an undesirable medical state, do not trigger an alarm and increases a risk of being overlooked.

Perhaps more concerningly, if a measure of proximity indicates that the values are close to the alarm threshold (without breaching it), then this may indicate that the alarm threshold is too close to value(s) of the physiological parameter. In particular, this may indicate that the physiological parameter has disadvantageously increased or decreased without activating the alarm, indicating that there is a risk that that the alarm threshold is inappropriately set (e.g., is set too high or too low).

The step of monitoring the physiological parameter may comprise receiving the set of values of the physiological parameter, e.g., from a medical sensing device or memory.

The one or more predetermined criteria may include a criterion that the measure of proximity breaches a first predetermined proximity threshold. This approach advantageously only recommends updating the alarm threshold when a threshold is breached. This provides a suitable mechanism for discriminating between when the alarm threshold should be updated and otherwise.

In some examples, a difference between the first predetermined proximity threshold and the alarm threshold is a predetermined percentage of a first predetermined range for the physiological parameter. This approach is particularly advantageous when the measure of proximity is an (average) measure of absolute distance between the set of values and the alarm threshold. More particularly, the first predetermined proximity threshold is thereby able to move with a change in the range for the physiological parameter to provide a relative threshold against which the measure of proximity can be compared.

The measure of proximity may be determined by: for each value in the set of values of the physiological parameter, determining an initial measure of proximity between the value and the alarm threshold, to thereby produce a set of initial measures of proximity; and averaging the set of initial measures of proximity to produce the measure of proximity.

In some examples, each initial measure of proximity is a relative measure of proximity of the value to the alarm threshold with respect to a second predetermined range for the physiological parameter. This advantageously allows the proposed approach to be deployed for any form of physiological parameter using, for example, the same criteria/criterion. In particular, this approach avoids a need to determine criteria for an absolute measure of proximity that may change between different individuals and/or use-case scenarios.

The second predetermined range may be defined by an upper alarm threshold, defining an upper bound for the range, and a lower alarm threshold, defining a lower bound for the range.

The measure of proximity may be determined by calculating how many of the values, in the set of values of the physiological parameter, breach an auxiliary threshold defined by the alarm threshold. This provides a mechanism for effectively identifying an average proximity of the physiological parameter to the alarm threshold, thereby being less sensitive to noise or error in the measuring of the physiological parameter.

In some examples, each value in the set of values of the physiological parameter is temporally adjacent to at least one other value in the set of values of the physiological parameter. In this way, the measure of proximity effectively represents a particular window of time of the physiological parameter.

The monitoring of the physiological parameter may comprise iteratively updating the measure of proximity, wherein, in each iteration of updating the measure of proximity, the set of values of the physiological parameter corresponds to a temporally later period of time than any previous iteration. This approach allows the physiological parameter to be iteratively monitored over a period of time. It will be appreciated that the set of values used in later iterations may comprise at least some of the values used in previous iterations, i.e., the windows of time represented by each set of values may overlap.

The one or more predetermined criteria may include a criterion that the measure of proximity has breached a second predetermined proximity threshold for no less than a predetermined period of time and/or a predetermined number of iterations of updating the measure of proximity. This approach is less sensitive to noise or error in monitoring of the physiological parameter, e.g., if there is a sudden spike in the physiological parameter that may be attributable to other factors (e.g., movement or the like).

In some examples, the computer-implemented method further comprises providing a user-perceptible output responsive to the update indicator. This provides an operator of a system making use of the alarm threshold with valuable information for determining or establishing whether or not the alarm threshold is to be updated and/or is out of date.

There is also provided a computer-implemented method for determining whether to adjust an alarm threshold for a physiological parameter, the computer-implemented method comprising: monitoring the alarm threshold for the physiological parameter; and responsive to the alarm threshold changing, performing any herein disclosed/proposed method. This approach thereby triggers the performance of a herein proposed method (only) when the alarm threshold changes. This advantageously avoids a need to continually check whether the alarm threshold needs to be adjusted, rather only reacting to a circumstance in which the alarm threshold is more likely to need subsequent adjustment.

There is also provided a computer-implemented method for determining whether to adjust a first alarm threshold and a second alarm threshold for a physiological parameter. The computer-implemented method comprises: monitoring a difference between the first alarm threshold and the second alarm threshold for the physiological parameter; and responsive to the monitored difference increasing: performing a herein disclosed approach for the first alarm threshold; and performing a herein disclosed approach for the second alarm threshold.

There is also provided a computer program product comprising computer program code means which, when executed on a computing device having a processing system, cause the processing system to perform all of the steps of any herein disclosed method.

There is also provided a processing system for determining whether to adjust an alarm threshold for a physiological parameter.

The processing system is configured to monitor a physiological parameter to determine a measure of proximity between a set of values of the physiological parameter and the alarm threshold for the physiological parameter; and responsive to the measure of proximity meeting one or more predetermined criteria, generate an update indicator that indicates that it is recommended to update the alarm threshold.

The processing system may be adapted to carry out any herein disclosed (computer-implemented) method. Similarly, the skilled person would be readily capable of defining a (computer-implemented) method for performing the functions of any herein disclosed processing system.

These and other aspects of the invention will be apparent from and elucidated with reference to the embodiment(s) described hereinafter.

## BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system in which embodiments can be employed;
Fig. 2 is a flowchart illustrating a proposed method;
Fig. 3 illustrates a mechanism for determining a measure of proximity;
Fig. 4 illustrates another mechanism for determining a measure of proximity;
Fig. 5 illustrates yet another mechanism for determining a measure of proximity;
Fig. 6 illustrates yet another mechanism for determining a measure of proximity;
Fig. 7 illustrates an alarm range;
Fig. 8 illustrates a mechanism for determining a measure of proximity;
Fig. 9 illustrates a mechanism for determining a measure of proximity;
Fig. 10 is a flowchart illustrating another proposed method; and
Fig. 11 is a flowchart illustrating yet another proposed method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for predicting whether or not an alarm threshold would benefit from being updated, i.e., is out of date. The physiological parameter, associated with the alarm threshold, is monitored and a set of values of the physiological parameter is processed to produce a proximity measure. Responsive to the proximity measure meeting one or more certain criteria, an update indicator indicating the alarm threshold should be updated is produced.

In the context of the present disclosure, a physiological parameter is any measurable or quantifiable parameter of a (e.g., medical) subject that is indicative of a physiological state or physiological information of the subject. Examples include vital sign parameters, but may be extended to include any parameter derivable from information gathered by monitoring a (e.g., medical) subject.

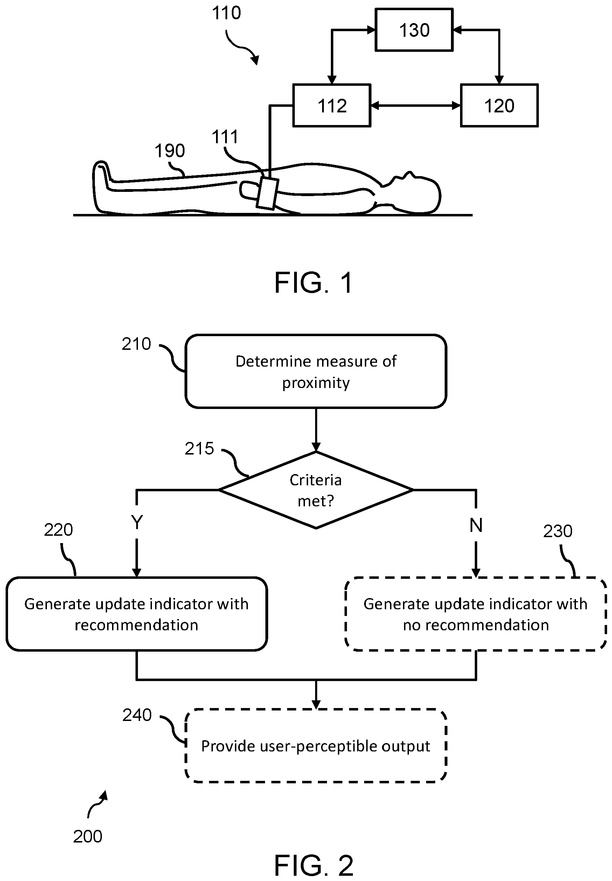

Fig. 1 illustrates a system 100 in which embodiments may be employed. The system 100 comprises a subject monitoring system 110 and an alarm system 120.

The subject monitoring system 110 is configured to monitor one or more physiological parameters of a subject 190, e.g., generate physiological data of the subject 190. The physiological data comprises, for each of the one or more physiological parameters, a respective value or measure, known as a parameter value, of the physiological parameter. Each respective value is continually or iteratively updated and effectively represents a sample of the measured physiological parameter.

The subject monitor system 110 may, for instance, comprise one or more subject sensors 111 configured to obtain sensor data of the subject. The subject monitor system may further comprise a sensor processing system 112 configured to receive and process the sensor data to generate the physiological data, i.e., monitor the physiological parameter(s), of the subject 190.

Example physiological parameters include those directly measurable from the subject, such as: SpO2; heart rate; respiratory rate; blood pressure; body temperature; blood glucose level; concentration of blood constituents (e.g., $HBO_2$ or HB) and so on.

Some examples of physiological parameters are produced by combining other physiological parameters and/or are derived by otherwise processing one or more other physiological parameters. Examples may include, for instance, a hemodynamic parameter (e.g., as suggested by the International Patent Application having publication number WO 2021/250224 A1), a respiratory instability (e.g., as suggested by the International Patent Application having publication number WO 2020/156909 A1) or a mortality risk (e.g., as suggested by the US patent application having publication number US 2015/213227 A1).

A wide variety of other examples of physiological parameters will be readily apparent to the skilled person. Suitable monitoring systems for monitoring physiological parameters are widely known and used in the art.

The alarm system 120 is configured to receive the physiological data of the subject, i.e., receive parameter values for physiological parameters. The alarm system 120 may be configured to compare each parameter value to one or more respective alarm thresholds. An alarm threshold can either be an upper alarm threshold or a lower alarm threshold. Responsive to the parameter value breaching a respective alarm threshold, the alarm system triggers an alarm. The parameter value breaches an upper alarm threshold when the parameter value exceeds the upper alarm threshold. The parameter value breaches a lower alarm threshold when the parameter value falls below the lower alarm threshold.

Triggering an alarm may, for instance, comprise generating a user-perceptible alert - such as a flashing light and/or an audible sound such as a siren. Of course, the alarm system 120 may, when triggering an alarm, provide a user-perceptible output such as a display providing information about the alarm, e.g., identifying which physiological parameter caused the triggering of the alarm.

Any alarm threshold may be defined manually (by a clinician) and/or automatically, e.g., to have a default value and/or to align with a value for a particular medical treatment. Of course, it is possible for any alarm threshold to be updated or modified manually and/or automatically.

The system 100 may, for instance, be integrated together in a patient monitor.

The present disclosure recognizes that misconfigured or otherwise inappropriate alarm thresholds for physiological parameters lead to negative outcomes for the subject and/or clinician responsible for the subject. For instance, if an upper alarm threshold is set too low and/or a lower alarm threshold set too high, then alarms may continually be generated. Similarly, if an upper alarm threshold is set too high and/or a lower alarm threshold too low, then alarms may fail to be generated even when a condition of the subject has deteriorated to a hazardous level.

Inappropriate thresholds may, for instance, be defined or set as a result of human error and/or during the course of certain medical procedures (e.g., provision of a medication or treatment known to affect a physiological parameter).

By way of example, during cerebral perfusion, a subject's blood pressure needs to be raised to a high value. The blood pressure value will undulate around this high value for a given time period. In such cases, an upper alarm threshold and lower alarm threshold for blood pressure may be intentionally increased, e.g., for use as guide rails to monitor the subject. If the upper alarm threshold is not reduced when the cerebral perfusion procedure has ended, this can lead to missed alarms during further monitoring of the subject.

The present disclosure provides a mechanism for identifying or determining whether to adjust an alarm threshold for a physiological parameter. The proposed approach uses a measure of proximity between a set of values for the physiological parameter and the alarm threshold to make the determination as to whether the alarm threshold needs to be updated.

One principle behind the proposed approach is that if a value of a physiological parameter moves closer towards one of the alarm thresholds without crossing it, e.g., and remains there for period of time, this would mean it is time for the alarm threshold(s) to be updated.

Embodiments may therefore provide a processing system 130 configured to determine whether to adjust an alarm threshold. The processing system may be communicatively coupled to the subject monitoring system and/or the alarm system, or formed as an aspect of one or both of these systems.

Fig. 2 is a flowchart illustrating a computer-implemented method 200 according to a proposed approach. The computer-implemented method 200 may be carried out by the processing system previously described, and is designed for determining whether to adjust an alarm threshold for a physiological parameter.

The method 200 comprises a step 210 of monitoring a physiological parameter to determine a measure of proximity between a set of values of the physiological parameter and the alarm threshold for the physiological parameter. Specific example approaches for determining such a measure of proximity are provided later in this disclosure.

In preferred examples, each value in the set of values of the physiological parameter is temporally adjacent to at least one other value in the set of values of the physiological parameter. In other words, the set of values may comprise a set of consecutive values of the physiological parameter.

The number of values in the set of values is no less than 2, e.g., no less than 5, e.g., no less than 10. The greater the number of values in the set of values, the less sensitive the proposed method is to noise (e.g., in the physiological parameter). However, too many values will waste resources (e.g., power or processing resource) whilst also, e.g., if the method is performed live, reducing a reactivity of the method to an ongoing circumstance. Thus, in some examples, the set of values may comprise no more than 1,000 values, e.g., no more than 500 values.

The method 200 may further comprise, responsive to the measure of proximity meeting one or more predetermined criteria, generating 220 an update indicator that indicates that it is recommended to update the alarm threshold. Otherwise, the method 200 may comprise performing a step 230 of generating an update indicator that indicates that it is not recommended to update the alarm threshold.

Thus, the method 200 may effectively comprise a determination step 215 of determining whether or not the measure of proximity meets one or more predetermined criteria. Responsive to a positive determination in step 215 (i.e., the one or more predetermined criteria are met), the method moves to step 220. Otherwise, the method performs step 230.

In this way, a recommendation as to whether or not an alarm threshold should be updated is responsive to a measure of proximity between multiple values of the physiological parameter and an alarm threshold.

It will be appreciated that the precise criterion/criteria used to determine whether or not step 220 is performed (i.e., in step 215) may vary dependent upon the precise nature of the measure of proximity.

However, in general, the one or more predetermined criteria may comprise a criterion that the measure of proximity breaches a first predetermined proximity threshold and/or that (if iteratively updated) the measure of proximity breaches the first predetermined proximity threshold for a predetermined period of time and/or number of iterations.

The method 200 may comprise a step 240 of comprising providing a user-perceptible output responsive to the update indicator. This may, for instance, comprise controlling a user interface to provide a visually-perceptible output (e.g., a flashing light or a display) responsive to the update indicator, e.g., to prompt a clinician or user that the alarm threshold(s) should be updated.

A first scenario is hereafter described in which the measure of proximity is determined by calculating how many of the values, in the set of values of the physiological parameter, breach an auxiliary threshold defined by the alarm threshold.

Where the alarm threshold is an upper alarm threshold, the auxiliary threshold is smaller than the alarm threshold and the measure of proximity for a set of values measures how many of the set of values exceed the auxiliary threshold.

Where the alarm threshold is a lower alarm threshold, the auxiliary threshold is greater than the alarm threshold and the measure of proximity for a set of values measures how many of the set of values fall below the auxiliary threshold.

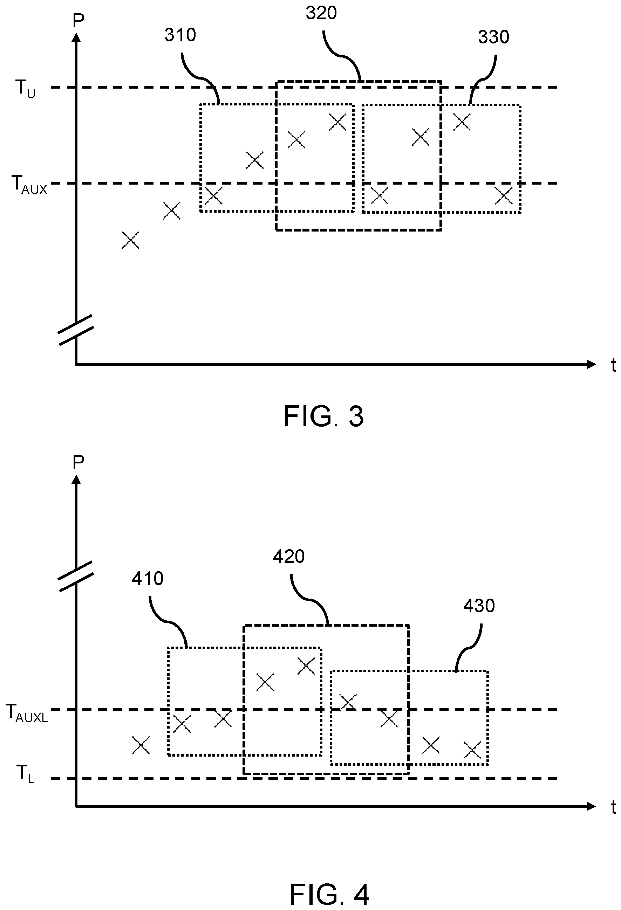

Fig. 3 illustrates one approach for determining the measure of proximity for different sets 310, 320, 330 of values according to the first scenario. In particular, Fig. 3 illustrates values for a physiological parameter P over time t. Each value is represented with a cross ("x").

Fig. 3 illustrates an alarm threshold Tu (here: an upper alarm threshold) and predetermined auxiliary threshold $T_{AUX}$. The measure of proximity counts, for a set of values, how many of the values breach (e.g., exceed) the predetermined auxiliary threshold $T_{AUX}$. Thus, in the illustrated example, a first set 310 of values is associated with a proximity value of 3, a second set 320 of values is associated with a proximity value of 3 and a third set 330 of values is associated with a proximity value of 2.

Fig. 4 illustrates another example of this same approach for determining the measure of proximity for different sets 410, 420, 430 of values according to the first scenario. In particular, Fig. 3 illustrates values for a physiological parameter P over time t. Each value is represented with a cross ("x").

Fig. 4 illustrates another alarm threshold $T_L$ (here: a lower alarm threshold) and predetermined auxiliary threshold $T_{AUXL}$. The measure of proximity counts, for a set of values, how many of the values breach (here: fall below) the predetermined auxiliary threshold $T_{AUX}$. Thus, in the illustrated example, a first set 410 of values is associated with a proximity value of 2, a second set 420 of values is associated with a proximity value of 1 and a third set 430 of values is associated with a proximity value of 3.

The auxiliary threshold $T_{AUX}$ is defined by the alarm threshold $T_u$ for the physiological parameter P. Example approaches for defining an auxiliary threshold $T_{AUX}$ are provided later in this disclosure.

In one variation of this first scenario, the one or more predetermined criteria may include a criterion that the measure of proximity breaches a predetermined proximity threshold. In this variation, the predetermined proximity threshold thereby represents a minimum number of times that the set of values should breach (here: exceed) the auxiliary threshold before a recommendation to update the alarm threshold is generated. For this variation, the predetermined proximity threshold may, for instance, be a predetermined fraction or percentage of the number of values in the set of values (e.g., no less than 75% of the set of values).

In another variation of this first scenario, performable when the measure of proximity is iteratively updated, the one or more predetermined criteria may include a criterion that the measure of proximity breaches (here: exceeds) a predetermined proximity threshold for a predetermined period of time and/or number of iterations (of updating the measure of proximity for different, later sets of values). The predetermined proximity threshold may be as previously indicated.

A wide variety of other variations for the first scenario will be apparent to the skilled person.

A second scenario is hereafter described in which the measure of proximity is determined by calculating a cumulative time that the (iteratively updated) value or moving average value of the physiological parameter breaches the auxiliary threshold. This measure of proximity thereby effectively indicates a length of time that a proximity condition for a set of values is met.

Similarly, the previously described embodiment, where the alarm threshold is an upper alarm threshold the auxiliary threshold is smaller than the alarm threshold and the measure of proximity for a set of values measures a (cumulative) length of time that the value or moving average value of the physiological parameter exceeds the auxiliary threshold.

Correspondingly, where the alarm threshold is a lower alarm threshold, the auxiliary threshold is greater than the alarm threshold and the measure of proximity for a set of values measures a (cumulative) length of time that the value or moving average value of the physiological parameter is below the auxiliary threshold.

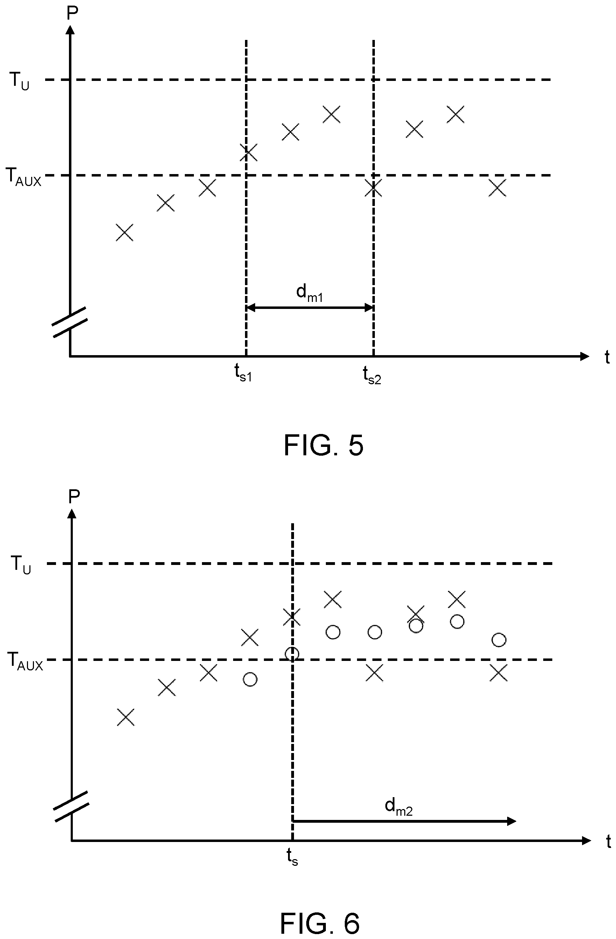

Fig. 5 illustrates one approach for determining the measure of proximity for a set of values according to the second scenario. In particular, Fig. 5 illustrates values for a physiological parameter P over time t. Each value is represented with a cross ("x").

Fig. 5 illustrates an alarm threshold Tu (here: an upper alarm threshold) and predetermined auxiliary threshold $T_{AUX}$. The measure of proximity $d_{m1}$ indicates for how long a value of the physiological parameter breaches the predetermined auxiliary threshold. This can be calculated, for instance, by monitoring the value of the physiological parameter and initiating a timer when the value breaches the auxiliary threshold (here: exceeds the auxiliary threshold) at a first time $t_{s1}$. The timer is then stopped when the value breaches the auxiliary threshold again (here: falls below the auxiliary threshold) at a second time $t_{s2}$. The value of the timer represents the proximity value for the set of values monitored during that time.

Fig. 6 illustrates an alternative approach for determining the measure of proximity for a set of values according to the second scenario. In particular, Fig. 6 illustrates values for a physiological parameter P over time t. Each value is represented with a cross ("x").

Fig. 6 also illustrates a moving average of values (here: averaging the 4 most recently obtained values with respect to a particular point in time). Each moving average is represented with a circle ("o").

Fig. 6 illustrates an alarm threshold Tu (here: an upper alarm threshold) and predetermined auxiliary threshold $T_{AUX}$. The measure of proximity $d_{m2}$ indicates for how long a moving average value of the physiological parameter breaches the predetermined auxiliary threshold. This can be calculated, for instance, by monitoring the moving average value of the physiological parameter and initiating a timer when the moving average value breaches the auxiliary threshold (here: exceeds the auxiliary threshold) at a first time $t_{s1}$. The timer is then stopped when the moving average value breaches the auxiliary threshold again (here: falls below the auxiliary threshold) at a second time $t_{s2}$. The value of the timer represents the proximity value for the set of moving values monitored during that time.

Use of a moving average makes the proximity value less sensitive to noise in the monitoring of the physiological parameter.

The auxiliary threshold $T_{AUX}$ is defined by the alarm threshold $T_U$ for the physiological parameter P. Example approaches for defining an auxiliary threshold $T_{AUX}$ are provided later in this disclosure.

In one variation of this second scenario, the one or more predetermined criteria may include a criterion that the measure of proximity breaches a predetermined proximity threshold. In this variation, the predetermined proximity threshold effectively represents a predetermined period of time or duration that the value (or moving average value) of the physiological value should remain above the auxiliary threshold before a recommendation to update the alarm is produced.

A wide variety of other variations for the second scenario will be apparent to the skilled person.

The first and second scenarios make use of an auxiliary threshold that is defined with respect to an alarm threshold. The auxiliary threshold may effectively represent the boundary or value at which the value of the physiological parameter is considered to be proximate or close to the alarm threshold.

Some described approaches for defining an auxiliary threshold make use of a range for the physiological parameter. This range may define an alarm range, e.g., bounded by an upper alarm threshold and a lower alarm threshold for the physiological parameter. The auxiliary threshold is definable with respect to one of the upper and lower alarm thresholds (representing the alarm threshold) and the alarm range. In particular, some approaches define the auxiliary threshold using a size of the alarm range, which is calculatable by subtracting the lower alarm threshold from the upper alarm threshold.

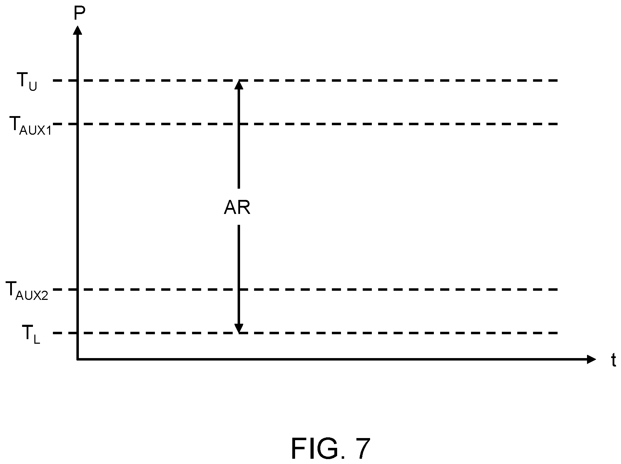

Fig. 7 illustrates an example of an alarm range AR for use in proposed embodiments. The alarm range spans between an upper alarm threshold Tu and a lower alarm threshold $T_L$. Fig. 7 also illustrates example auxiliary thresholds, a first auxiliary threshold $T_{AUX1}$ is defined with respect to the upper alarm threshold Tu (and the alarm range AR) and a second auxiliary threshold $T_{AUX2}$ is defined with respect to a lower alarm threshold $T_L$ (and the alarm range AR).

In some examples, the auxiliary threshold may be a predetermined multiple of the alarm threshold. For instance, where the alarm threshold is an upper threshold Tu, the auxiliary threshold may be set to be equal to a predefined scalar (e.g., 0.9) times the upper threshold.

In other examples, the auxiliary threshold may be a predetermined distance from the (corresponding) alarm threshold. The predetermined distance may be calculated by multiplying the size of the alarm range AR by a predetermined scalar. The auxiliary threshold is thereby positioned between the upper and lower alarm thresholds. The predetermined scalar may have a predetermined value, e.g., a non-zero value less than 0.25, e.g., 0.2, 0.15 or 0.1.

In some examples, the auxiliary threshold is updated responsive to a change in the alarm threshold. In preferred versions of such examples, the auxiliary threshold is configured to be relatively more distant from the alarm threshold (with respect to the alarm range) responsive to the alarm range increasing. This approach means that detection of unduly alarm ranges are more likely to be identified. For instance, if the alarm range increases by a factor of X (i.e., the new alarm range is X times the old alarm range), then the distance between the alarm threshold and the auxiliary threshold may increase by a factor of $X^Y$, where Y is any value greater than 1.

A third scenario is hereafter described in which the measure of proximity is determined by calculating a mean (i.e., average) of the distances between the values in the set of values and the alarm threshold.

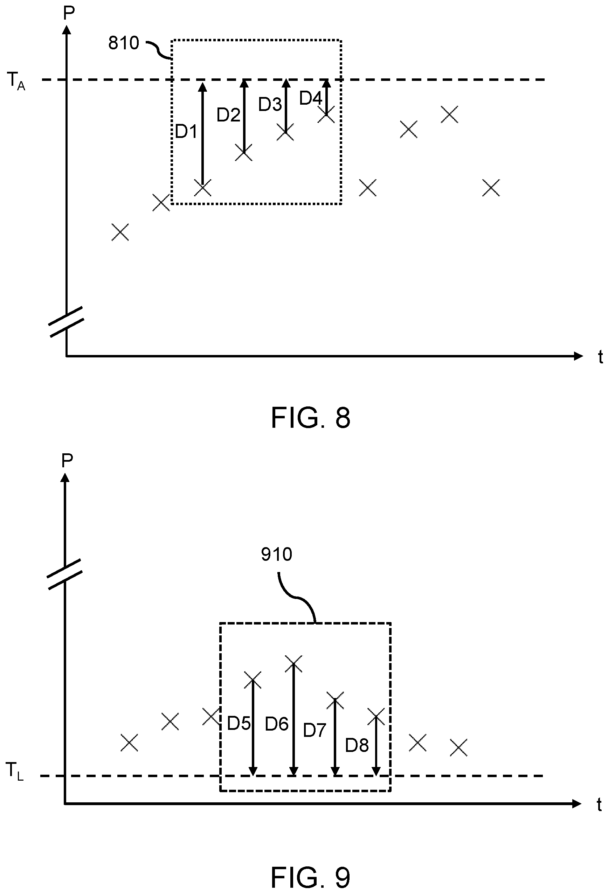

Figs 8 and 9 illustrates an example of one approach for determining the measure of proximity for a (respective) set 810, 910 of values according to the third scenario. In particular, Figs 8 and 9 both illustrate example values for a physiological parameter P over time t. Each value is represented with a cross ("x"). Fig. 8 illustrates an alarm threshold Tu embodied as an upper alarm threshold. Fig. 9 also illustrates an alarm threshold $T_L$, which is instead embodied as a lower alarm threshold.

In one variation, the measure of proximity may be determined by calculating a distance D1, D2, D3, D4, D5, D6, D7, D8 between each value (in the corresponding set of values 810, 910) and the corresponding alarm threshold Tu, $T_L$ and averaging the calculated distances. A distance can be trivially calculated by determining the modulus or absolute value of the alarm threshold minus the value of the physiological parameter.

Thus, in this variant, the measure of proximity M for a set of values can be determined using the following equation:

$$M = \frac{\sum_{i=1}^{j} |T_A - v_i|}{j} = \sum_{i=1}^{j} \frac{|T_A - v_i|}{j} \tag{1}$$

where: $|\cdot|$ indicates the absolute value of the enclosed number; $T_A$ is the alarm threshold (i.e., $T_U$ or $T_L$); $v_i$ represents the i-th value of the set of values (of the physiological parameter); and j indicates the total number of values in the set of values.

In this way, the measure of proximity is effectively calculated by, for each value in the set of values of the physiological parameter, determining an initial measure of proximity ($|T_A - v_i|$) between the value $v_i$ and the alarm threshold $T_A$, to thereby produce a set of initial measures of proximity. The initial measures of proximity are then averaged to produce the measure of proximity.

In another variation, the measure of proximity may be determined by calculating a relative measure of proximity for each value and averaging the relative measures of proximity. The relative measure of proximity may indicate a relative proximity of the value with respect to the size of the alarm range (previously described).

Thus, in this variation, the measure of proximity M for a set of values can be determined using the following equation:

$$M = \frac{\sum_{i=1}^{j} \frac{|T_A - v_i|}{(T_U - T_L)}}{j} \tag{2}$$

where $|\cdot|$ indicates the absolute value of the enclosed number, $T_A$ is the alarm threshold (being one of the upper and lower alarm thresholds, Tu is the upper alarm threshold, $T_L$ is the lower alarm threshold), $v_i$ represents the i-th value of the set of values and j indicates the total number of values in the set of values.

Thus, the measure of proximity calculated according to equation (1) effectively represents an absolute average distance between a set of values and the alarm threshold. The measure of proximity calculated according to equation (1) effectively represents a relative average distance between a set of values and the alarm threshold, specifically relative to the overall alarm range.

In one variation of this third scenario, the one or more predetermined criteria may include a criterion that the measure of proximity breaches (e.g., falls below) a predetermined proximity threshold. In this variation, the predetermined proximity threshold thereby represents a maximum average distance between the set of values and the alarm threshold before a recommendation to update the alarm threshold is generated. For this variation, the predetermined proximity threshold may, for instance, be a predetermined average distance.

For this variation, the predetermined proximity threshold may, for instance, be a predefined number (e.g., a value less than 0.1).

In some examples, the predetermined proximity threshold may change responsive to changes in the alarm range, e.g., proportionally to the alarm range. This means detection of unduly alarm ranges are more likely to be identified.

In one example, if the alarm range increases by a factor of X, then the predetermined proximity threshold may increase by Q.X (i.e., Q times X). Q should have a value less than 1, e.g., a value of between 0.05 and 0.4, e.g., a value of 0.2.

Table 1 illustrates example changes to a predetermined proximity threshold $TH_{PD}$ responsive to a change in the alarm range, specifically a scaling factor SF of the alarm range (which represents a ratio between the (new) alarm range and the previous/default alarm range). In this example, the value of Q is 0.2.

TABLE 1

| Previous/Default Proximity Threshold | Scaling Factor (SF) | Proximity Threshold ($TH_{PD}$) |
|---|---|---|
| 0.1 | 1 | 0.1 |
| 0.1 | 1.2 | 0.34 |
| 0.1 | 1.4 | 0.38 |
| 0.1 | 1.6 | 0.42 |
| 0.1 | 1.8 | 0.46 |

(continued)

| Previous/Default Proximity Threshold | Scaling Factor (SF) | Proximity Threshold ($TH_{PD}$) |
|---|---|---|
| 0.1 | 2.0 | 0.50 |

As demonstrated in Table 1, in some examples, no change is made to the predetermined proximity threshold $TH_{PD}$ if there is no change in the alarm range (i.e., the scaling factor is 1).

In one variation of this third scenario, the one or more predetermined criteria may include a criterion that the measure of proximity breaches (e.g., exceeds) a second predetermined proximity threshold. In this variation, the second predetermined proximity threshold thereby represents a minimum average distance between the set of values and the alarm threshold before a recommendation to update the alarm threshold is generated.

In this approach, the second predetermined proximity threshold may represent a threshold close to an opposite end of the relative scale. In particular, due to the application of equation (1) or (2), the measure of proximity represents a relative measure of distance (e.g., on a scale of 0 to 1) within an alarm range. The second predetermined threshold may effectively indicate an average proximity to a furthest side of the alarm range.

For this variation, the second predetermined proximity threshold may, for instance, be a predefined number (e.g., a value equal to 0.9).

In some examples, the second predetermined proximity threshold may change responsive to changes in the alarm range, e.g., proportionally to the alarm range. This means detection of unduly alarm ranges are more likely to be identified.

In one example, if the alarm range increases by a factor of X, then the predetermined proximity threshold may be reduced by Z.X (i.e., Z times X). Z should have a value less than 1, e.g., a value of between 0.05 and 0.4, e.g., a value of 0.2.

Table 2 illustrates example changes to a second predetermined proximity threshold $TH_{PD2}$ responsive to a change in the alarm range, specifically a scaling factor SF of the alarm range (which represents a ratio between the (new) alarm range and the previous/default alarm range). In this example, the value of Z is 0.2.

TABLE 2

| Previous/Default Proximity Threshold | Scaling Factor (SF) | 2nd Proximity Threshold ($TH_{PD2}$) |
|---|---|---|
| 0.9 | 1 | 0.9 |
| 0.9 | 1.2 | 0.66 |
| 0.9 | 1.4 | 0.62 |
| 0.9 | 1.6 | 0.58 |
| 0.9 | 1.8 | 0.54 |
| 0.9 | 2.0 | 0.5 |

As demonstrated in Table 2, in some examples, no change is made to the second predetermined proximity threshold $TH_{PD2}$ if there is no change in the alarm range (i.e., the scaling factor is 1).

In another variation of this third scenario, performable when the measure of proximity is iteratively updated, the one or more predetermined criteria may include a criterion that the measure of proximity breaches (here: falls below) the predetermined proximity threshold for (no less than) a predetermined period of time and/or number of iterations (of updating the measure of proximity for different, later sets of values). The predetermined proximity threshold may be as previously indicated.

In another variation of this third scenario, performable when the measure of proximity is iteratively updated, the one or more predetermined criteria may include a criterion that the measure of proximity breaches (here: exceeds) the second predetermined proximity threshold for (no less than) a predetermined period of time and/or number of iterations (of updating the measure of proximity for different, later sets of values). The second predetermined proximity threshold may be as previously indicated.

A wide variety of other variations for the third scenario will be apparent to the skilled person.

A number of previously described embodiments may use of a predetermined period of time for use in the one or more predetermined criteria for determining whether or not to recommend that an alarm threshold should be updated.

In some examples, the predetermined period of time is a predetermined duration. For instance, the predetermined duration may have a value of 10 minutes. In preferred such examples, the predetermined duration is dependent upon the physiological parameter, e.g., changes responsive to the (type of) physiological parameter that is being monitored.

In further embodiments, the predetermined period of time is updated responsive to changes in the size of the alarm range, e.g., inversely proportional to changes in the alarm range. Thus, the predetermined period of time $T_{PD}$ may be updated according to the following equation:

$$T_{PD} = \frac{ARO.T_{PDO}}{AR} \qquad\qquad (3)$$

where $T_{PD}$ is the updated predetermined period of time, ARO is the size of the previous or old alarm range, $T_{PDO}$ is the previous or old predetermined period of time and AR is the size of the update alarm range. Thus, the value AR/ARO represents the scalar change to the size of the alarm range.

The predetermined period of time may be reset to the predetermined duration. The update to the predetermined period of time $T_{PD}$ may be performed automatically, for instance, responsive to the alarm threshold being modified after a recommendation to update the alarm threshold has been generated and/or manually, for instance, responsive to a corresponding user input.

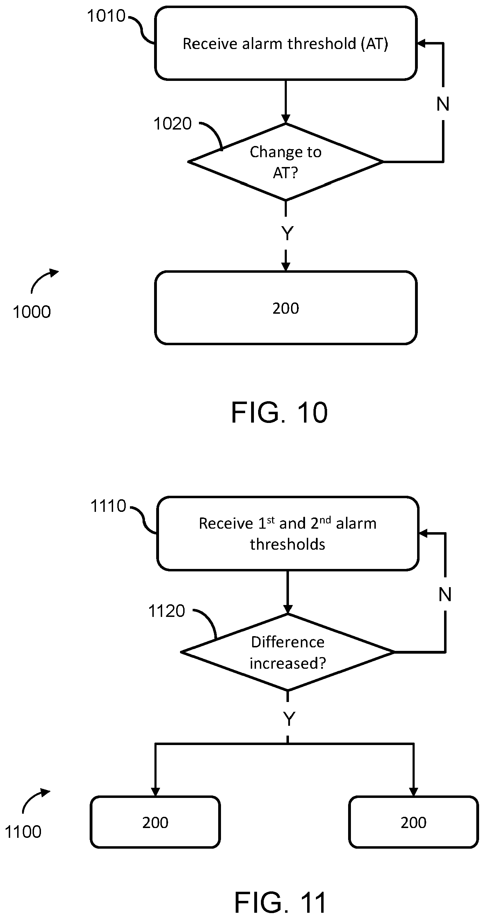

Fig. 10 illustrates another herein proposed computer-implemented method 1000. The method 1000 is configured for determining whether to adjust an alarm threshold for a physiological parameter.

The method 1000 comprises selectively performing the method 200 previously explained. In particular, the method 1000 may comprise monitoring 1010, 1020 the alarm threshold for the physiological parameter. The method 200 is only performed when the alarm threshold has changed. In this way, the method 200 is performed only when there is a risk that the alarm threshold is inappropriate, e.g., due to being changed.

The monitoring 1010, 1020 may comprise a step 1010 of receiving the alarm threshold and a step 1020 of determining whether or not the alarm threshold has changed (compared to a previously obtained alarm threshold). Responsive to a positive determination (i.e., the alarm threshold has changed), then the method 1000 performs method 200. Otherwise, the method reverts back to step 1010 and a new alarm threshold is obtained.

In another example, the monitoring 1010, 1020 comprises receiving an indicator that indicates whether or not the alarm threshold has changed. This may, for instance, take the form of a signal that indicates an intention to change the alarm threshold, e.g., responsive to a user input and/or automatically.

In some examples, method 200 is performed and/or iteratively repeated, e.g., until a predetermined time period has elapsed and/or (if relevant) a predetermined number of iterations of method 200 have been performed. This approach avoids continual performance of method 200, which may waste resource. The predetermined time period may, for instance, be no less than 1 hour, e.g., no less than 6 hours.

Fig. 11 illustrates another herein proposed computer-implemented method 1100. The method 1100 is configured for determining whether to adjust a first alarm threshold (e.g., an upper alarm threshold) and a second alarm threshold (e.g. a lower alarm threshold) for a physiological parameter.

The method 1100 comprises selectively performing two different instances of the method 200 previously explained.

In particular, the method 1100 may comprise monitoring 1110, 1120 monitoring a difference between the first alarm threshold and the second alarm threshold for the physiological parameter. Each instance of method 200 is only performed responsive to the monitored difference increasing. In this way, the instances of method 200 is performed only when there is a risk that the alarm threshold is inappropriate, e.g., due to being changed.

The monitoring 1110, 1120 may comprise a step 1110 of receiving the first alarm threshold and the second alarm threshold and a step 1120 of determining whether or not the difference between the two alarm thresholds has increased (compared to a previously obtained difference). Responsive to a positive determination (i.e., the difference has increased), then the method performs an instance of step 200 for each alarm threshold. Otherwise, the method reverts back to step 1110 and a new first and second alarm thresholds are obtained.

In another example, the monitoring 1110, 1120 comprises receiving an indicator that indicates whether or not the difference between the first and second alarm thresholds has increased. This may, for instance, take the form of a signal that indicates an intention to change one of the alarm thresholds, e.g., responsive to a user input and/or automatically.

In some examples, each instance of method 200 is performed and/or iteratively repeated, e.g., until a predetermined time period has elapsed and/or (if relevant) a predetermined number of iterations of each instance of method 200 have been performed. This approach avoids continual performance of the instances of method 200, which may waste resource. The predetermined time period may, for instance, be no less than 1 hour, e.g., no less than 6 hours.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method (200) for determining whether to adjust an alarm threshold ($T_u$, $T_L$) for a physiological parameter, the computer-implemented method comprising:

   monitoring (210) a physiological parameter to determine a measure of proximity between a set of values (310, 320, 330, 410, 420, 430, 810, 910) of the physiological parameter and the alarm threshold for the physiological parameter; and
   responsive to the measure of proximity meeting one or more predetermined criteria, generating (220) an update indicator that indicates that it is recommended to update the alarm threshold.

2. The computer-implemented method (200) of claim 1, wherein the one or more predetermined criteria includes a criterion that the measure of proximity breaches a first predetermined proximity threshold.

3. The computer-implemented method (200) of claim 2, wherein a difference between the first predetermined proximity threshold and the alarm threshold is a predetermined percentage of a first predetermined range for the physiological parameter.

4. The computer-implemented method (200) of any one of claims 1 to 3, wherein the measure of proximity is determined by:

   for each value in the set of values (810, 910) of the physiological parameter, determining an initial measure of proximity (D1, D2, D3, D4, D5, D6, D7, D8) between the value and the alarm threshold, to thereby produce a set of initial measures of proximity; and
averaging the set of initial measures of proximity to produce the measure of proximity.

5. The computer-implemented method (200) of claim 4, wherein each initial measure of proximity is a relative measure of proximity of the value to the alarm threshold with respect to a second predetermined range for the physiological parameter.

6. The computer-implemented method (200) of claim 5, wherein the second predetermined range is defined by an upper alarm threshold (Tu), defining an upper bound for the range, and a lower alarm threshold ($T_L$), defining a lower bound for the range.

7. The computer-implemented method (200) of any one of claims 1 to 2, wherein the measure of proximity is determined by calculating how many of the values, in the set of values (310, 320, 330, 410, 420, 430) of the physiological parameter, breach an auxiliary threshold defined by the alarm threshold.

8. The computer-implemented method (200) of any one of claims 1 to 7, wherein each value in the set of values of the physiological parameter is temporally adj acent to at least one other value in the set of values of the physiological parameter.

9. The computer-implemented method (200) of any of claims 1 to 8, wherein the monitoring the physiological parameter comprises iteratively updating the measure of proximity,
wherein, in each iteration of updating the measure of proximity, the set of values of the physiological parameter corresponds to a temporally later period of time than any previous iteration.

10. The computer-implemented method (200) of claim 9, wherein the one or more predetermined criteria includes a criterion that the measure of proximity has breached a second predetermined proximity threshold for no less than a predetermined period of time and/or a predetermined number of iterations of updating the measure of proximity.

11. The computer-implemented method (200) of any one of claims 1 to 10, further comprising providing (240) a user-perceptible output responsive to the update indicator.

12. A computer-implemented method (1000) for determining whether to adjust an alarm threshold for a physiological parameter, the computer-implemented method comprising:

monitoring (1010, 1020) the alarm threshold for the physiological parameter; and
responsive to the alarm threshold changing, performing the method (200) of any one of claims 1 to 11.

13. A computer-implemented method (1100) for determining whether to adjust a first alarm threshold and a second alarm threshold for a physiological parameter, the computer-implemented method comprising:

monitoring (1110, 1120) a difference between the first alarm threshold and the second alarm threshold for the physiological parameter; and
responsive to the monitored difference increasing:

performing the method (200) of any one of claims 1 to 11 for the first alarm threshold; and
performing the method (200) of any one of claims 1 to 11 for the second alarm threshold.

14. A computer program product comprising computer program code means which, when executed on a computing device having a processing system, cause the processing system to perform all of the steps of the method according to any of claims 1 to 13.

15. A processing system for determining whether to adjust an alarm threshold for a physiological parameter, the processing system being configured to:

monitor a physiological parameter to determine a measure of proximity between a set of values of the physiological parameter and the alarm threshold for the physiological parameter; and
responsive to the measure of proximity meeting one or more predetermined criteria, generate an update indicator that indicates that it is recommended to update the alarm threshold.

110

130

112 ⟷ 120

111

190

FIG. 1

210 — Determine measure of proximity

215 — Criteria met?

Y

N

220 — Generate update indicator with recommendation

230 — Generate update indicator with no recommendation

240 — Provide user-perceptible output

200

FIG. 2

FIG. 3

FIG. 4

FIG. 5

FIG. 6

FIG. 7

FIG. 8

FIG. 9

FIG. 10

FIG. 11

EUROPEAN SEARCH REPORT

Application Number

EP 24 18 5586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/158550 A1 (WATSON JAMES [GB] ET AL) 7 June 2018 (2018-06-07) * paragraph [0027]; claim 1 * ----- | 1-15 | INV. G16H40/63 |
| A | CN 116 369 849 A (SHENZHEN MINDRAY BIOMEDICAL ELECTRONICS CO LTD) 4 July 2023 (2023-07-04) * the whole document * ----- | 1-15 | |

**TECHNICAL FIELDS SEARCHED (IPC)**

G16H
A61B

The present search report has been drawn up for all claims

| Place of search | Date of completion of the search | Examiner |
|---|---|---|
| Munich | 24 January 2025 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 5586

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2025

| Patent document cited in search report | Publication date | Patent family member(s) | Publication date |
|---|---|---|---|
| US 2018158550 A1 | 07-06-2018 | US 2015363561 A1<br>US 2018158550 A1 | 17-12-2015<br>07-06-2018 |
| CN 116369849 A | 04-07-2023 | NONE | |

EPO FORM P0459

For more details about this annex : see Official Journal of the European Patent Office, No. 12/82

**EP 4 675 636 A1**

## REFERENCES CITED IN THE DESCRIPTION

**Patent documents cited in the description**

- WO 2021250224 A1
- WO 2020156909 A1
- US 2015213227 A1